# EUROPEAN PATENT SPECIFICATION

(11) **EP 3 087 381 B1**
(45) Date of publication and mention of the grant of the patent: **15.05.2019**
(21) Application number: 14816307.4
(22) Date of filing: 22.12.2014
(51) Int. Cl.: G01N 27/327

(54) **HAND-HELD TEST METER WITH AN OPERATING RANGE TEST STRIP SIMULATION CIRCUIT BLOCK**
TRAGBARE MESSVORRICHTUNG MIT EINEM SIMULATIONSSCHALTBLOCK FÜR TESTSTREIFEN IM BETRIEBSBEREICH
DISPOSITIF DE MESURE D'ANALYSE PORTABLE AYANT UN BLOC DE CIRCUITS DE SIMULATION DE BANDELETTE RÉACTIVE DE PLAGE FONCTIONNELLE

(30) Priority: 23.12.2013 GB 201322927
(43) Date of publication of application: 02.11.2016
(73) Proprietor: Lifescan Scotland Limited, Inverness IV2 3ED (GB)
(72) Inventor: LLOYD, Timothy, Inverness IV2 3ED (GB); MCCOLL, David, Inverness IV2 3ED (GB); MASSARI, Rossano, I-20126 MI (IT); FORLANI, Christian, I-20126 MI (IT)
(74) Representative: Brunner, John Michael Owen
(86) International application number: PCT/EP2014/079064
(87) International publication number: WO 2015/097179

(56) References cited:
- WO-A2-2009/036429
- US-A1- 2002 133 064
- US-A1- 2012 053 436
- US-B1- 6 645 368

## Description

### FIELD OF THE INVENTION

The present invention relates, in general, to medical devices and, in particular, to test meters and related methods.

### BACKGROUND OF THE INVENTION

The determination (e.g., detection and/or concentration measurement) of an analyte in, or characteristic of, a bodily fluid sample is of particular interest in the medical field. For example, it can be desirable to determine glucose, ketone bodies, cholesterol, lipoproteins, triglycerides, acetaminophen, haematocrit and/or HbA1c concentrations in a sample of a bodily fluid such as urine, blood, plasma or interstitial fluid. Such determinations can be achieved using a hand-held test meter in combination with analytical test strips (e.g., electrochemical-based analytical test strips).

A quality assurance system and method for a component as a blood testing cartridge including an electrochemical sensor is disclosed in WO2009/036429. The device described there comprises an electronic simulator which is configured to simulate signals produced by the component. The simulation signals are used for testing electrical integrity of an electrical connector or operational integrity of operational amplifiers of the test system.

### SUMMARY OF THE INVENTION

According to one aspect of the present invention there is provided a hand-held test meter for use with an electrochemical-based analytical test strip in the determination of an analyte in a bodily fluid sample. The hand-held test meter comprises a housing; a micro-controller disposed in the housing; an operating range test strip simulation circuit block disposed in the housing; and a strip port connector configured to operationally receive an electrochemical-based analytical test strip. The operating range test strip simulation circuit block is in electrical communication with the strip port connector; and the operating range test strip simulation circuit block is configured to simulate an electrochemical-based analytical test strip inserted into the strip port connector and an operating range of bodily fluid samples applied to the electrochemical-based analytical test strip by sequentially presenting a plurality of electrical loads. Each of the plurality of electrical loads is configured as a first resistor of predetermined value in series with a parallel configuration of a second resistor of predetermined value and a first capacitor of predetermined value. The strip port connector is configured in electrical communication with the micro-controller.

The test strip and applied bodily fluid sample circuit block of the hand held test meter may be configured to simulate a plurality of applied bodily fluid samples across either a glucose operating range of the hand-held test meter, a hematocrit operating range of the hand-held test meter or a combined glucose and hematocrit operating range of the hand-held test meter. The test strip and applied bodily fluid sample circuit block may be the operating range test strip simulation circuit block.

In one embodiment, the hand-held test meter may comprise twelve electrical loads.

In any embodiment, the first resistor in each of the plurality of electrical loads in the hand-held test meter may be essentially identical.

The plurality of operating loads of the hand-held test meter may simulate an operating range that includes a design and manufacturing guard band which may be +/- 30%.

In one embodiment, the first resistor may have a predetermined value of 5,100 ohms, the second resistor may have a predetermined value in the range of 16,000 ohms to 390,000 ohms, and the first capacitor may have a predetermined value in the range of 0 pF to 6.2 pF or in the range of 0 pF to 8.2 pF.

In one embodiment, the plurality of electrical loads may share the first resistor.

The electrochemical-based analytical test strip of the hand-held test meter may be configured for the determination of glucose in a whole blood bodily fluid sample.

The operating range test strip simulation circuit block of the hand-held test meter may be further configured to simulate an electrochemical-based analytical test strip inserted into the strip port connector and an operating range of control solutions applied to the electrochemical-based analytical test strip by sequentially presenting the plurality of loads.

A second aspect of the present invention is a method for employing a hand-held test meter for use with an electrochemical-based analytical test strip in the determination of an analyte in, or a characteristic of, a bodily fluid sample. The method comprises employing an operating range test strip simulation circuit block of a hand-held test meter by activating the operating range test strip simulation circuit block; presenting in a sequential manner, to a port connector of the hand-held test meter by the operating range test strip simulation circuit block, upon the activating of the operating range test strip simulation circuit block, a plurality of electrical loads. Each of the plurality of electrical loads are configured as a first resistor of predetermined value in series with a parallel configuration of a second resistor of predetermined value, and a first capacitor of predetermined value. The plurality of electrical loads spans the working range of the hand-held test meter with respect to a predetermined bodily fluid sample and at least one of an analyte in the bodily fluid sample and characteristic of the bodily fluid sample.

The employing and presenting of the above method may serve to test operation of the hand-held test meter prior to use of the hand-held test meter for the determination of an analyte.

The method may further include the steps of inserting an electrochemical-based analytical test strip into the hand-held test meter following the presenting and subsequently determining at least one of an analyte in, and a characteristic of, a bodily fluid sample applied to the analytical test strip using a micro-controller of the hand-held test meter.

The test strip and applied bodily fluid sample circuit block may be configured to simulate a plurality of applied bodily fluid samples across a glucose operating range of the hand-held test meter, a hematocrit operating range of the hand-held test meter or a combined glucose and hematocrit operating range of the hand-held test meter.

The plurality of electrical loads of the above method may be twelve electrical loads.

The first resistor of in each of the plurality of electrical loads may be essentially identical.

The plurality of operating loads may simulate an operating range that includes a guard band, which may be +/- 30%.

The first resistor may have a predetermined value of 5,100 ohms, the second resistor may have a predetermined value in the range of 16,000 ohms to 390,000 ohms, and the first capacitor may have a predetermined value in the range of 0 pF to 6.2 pF.

The plurality of electrical loads may share the first resistor.

The electrochemical-based analytical test strip may be an electrochemical-based analytical test strip configured for the determination of glucose in a whole blood bodily fluid sample.

The operating range test strip simulation circuit block may be further configured to simulate an electrochemical-based analytical test strip inserted into the strip port connector and an operating range of control solutions applied to the electrochemical-based analytical test strip by sequentially presenting the plurality of loads.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings, in which like numerals indicate like elements, of which:
FIG. 1 is a simplified depiction of a hand-held test meter according to an embodiment of the present invention;
FIG. 2 is a simplified block diagram of various blocks of the hand-held test meter of FIG. 1;
FIG. 3 is a simplified schematic diagram of a single electrical load of an operating range test strip simulation circuit block as can be employed in embodiments of the present invention;
FIG. 4 is a simplified schematic of a configuration of a plurality of electrical loads (i.e., loads 1 through n, where n = any suitable number greater than 1 such as, for example, n = 12 as detailed with regard to Table 1 herein) as can be employed in embodiments of the present invention; and
FIG. 5 is a flow diagram depicting stages in a method for operating a hand-held test meter according to an embodiment of the present invention.

### DETAILED DESCRIPTION OF ILLUSTRATIVE EMBODIMENTS

The following detailed description should be read with reference to the drawings, in which like elements in different drawings are identically numbered. The drawings, which are not necessarily to scale, depict exemplary embodiments for the purpose of explanation only and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein.

In general, hand-held test meters for use with an electrochemical-based analytical test strip in the determination of an analyte (such as glucose) in, and/or a characteristic (for example, hematocrit) of, a bodily fluid sample (such as for example, a whole blood sample) according to embodiments of the present invention include a housing, a micro-controller disposed in the housing, an operating range test strip simulation circuit block disposed in the housing and a strip port connector configured to operationally receive an electrochemical-based analytical test strip. The operating range test strip simulation circuit block is in electrical communication with (for example, electrically connected in a direct or indirect manner) the strip port connector. In addition, the operating range test strip simulation circuit block is configured to simulate an electrochemical-based analytical test strip inserted into the strip port connector and an operating range of bodily fluid samples applied to such an electrochemical-based analytical test strip by sequentially presenting a plurality of electrical loads. Each of the plurality of electrical loads is configured as a first resistor in series with a parallel configuration of a second resistor and a first capacitor. Moreover, the strip port connector is configured in electrical communication with the micro-controller.

Hand-held test meters according to embodiments of the present invention are beneficial in that the operating range test strip simulation circuit block is configured such that a simulation of an entire operating range of an analyte in a bodily fluid sample (such as glucose in a whole blood sample) and/or a characteristic of an bodily fluid sample (for example, hematocrit of a whole blood sample) can be performed, thus fully testing proper operation of the hand-held test meter across the operating range. In some embodiments, the plurality of electrical loads also beneficially tests the operating range of control solutions conventionally employed to test operation of the hand-held test meter. In addition, such hand-held test meters are beneficial in that the operating range test strip simulation circuit block can be employed to easily and repeatedly test operation of the hand-held test meter without the need for, or the variation induced by, an actual electrochemical-based analytical test strip and a plurality of control solutions that mimic bodily fluid samples across the entire operating range of the hand-held test meter.

Once one skilled in the art is apprised of the present disclosure, he or she will recognize that an example of a hand-held test meter that can be readily modified as a hand-held test meter according to the present invention is the commercially available OneTouch® Ultra® 2 glucose meter from LifeScan Inc. (Milpitas, California). Additional examples of hand-held test meters that can also be modified are found in U.S. Patent Application Publications No's. 2007/0084734 (published on April 19, 2007) and 2007/0087397 (published on April 19, 2007) and in International Publication Number WO2010/049669 (published on May 6, 2010), and Great Britain Patent Application No. 1303616.5, filed on February 28, 2013, each of which is hereby incorporated herein in full by reference.

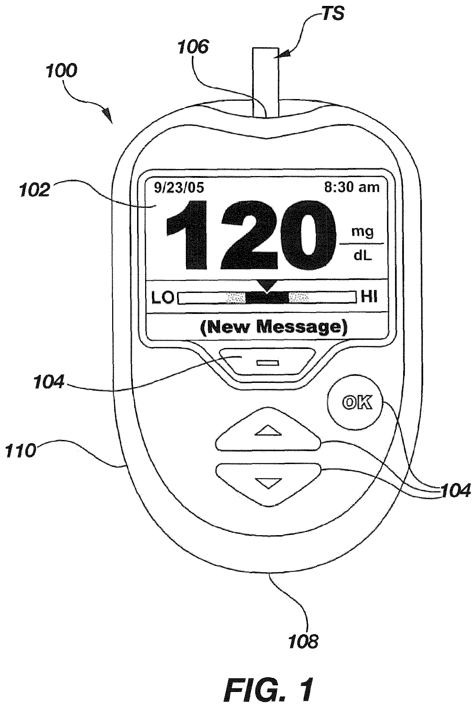

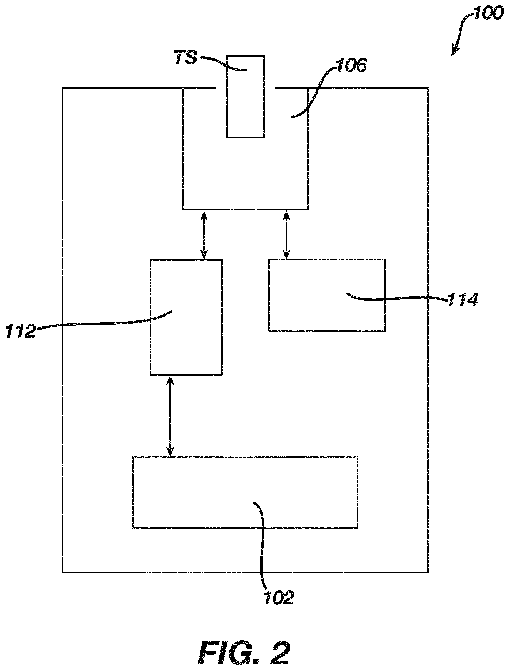

FIG. 1 is a simplified depiction of a hand-held test meter 100 for the determination of an analyte in, and/or a characteristic of, a bodily fluid sample according to an embodiment of the present invention. FIG. 2 is a simplified block diagram of various blocks of hand-held test meter 100.

Referring to FIGs. 1 and 2, hand-held test meter 100 includes a display 102, a plurality of user interface buttons 104, a strip port connector 106, a USB interface 108, and a housing 110 (see FIG. 1). Referring to FIG. 2 in particular, hand-held test meter 100 also includes a micro-controller block 112, an operating range test strip simulation circuit block 114, and other electronic components (not shown) for applying an electrical bias (e.g., an alternating current (AC) and/or direct current (DC) bias) to an electrochemical-based analytical test strip (labeled TS in FIGs. 1 and 2), and also for measuring an electrochemical response (e.g., plurality of test current values, phase, and/or magnitude) and determining an analyte or characteristic based on the electrochemical response. To simplify the current descriptions, the figures do not depict all such electronic circuitry.

Display 102 can be, for example, a liquid crystal display or a bi-stable display configured to show a screen image. An example of a screen image during the determination of an analyte in a bodily fluid sample may include a glucose concentration, a date and time, an error message, and a user interface for instructing a user how to perform a test. Examples of screen images during use of the operating range test strip simulation circuit block may be an image reporting that a hand-held test meter operating range test passed, or an image reporting that the hand-held test meter operating range test has resulted in an error.

Strip port connector 106 is configured to operatively interface with an electrochemical-based analytical test strip TS, such as an electrochemical-based analytical test strip configured for the determination of hematocrit and/or glucose in a whole blood sample. Therefore, the electrochemical-based analytical test strip is configured for operative insertion into strip port connector 106 and to operatively interface with micro-controller block 112 via, for example, suitable electrical contacts, wires, electrical interconnects or other structures known to one skilled in the art.

USB Interface 108 can be any suitable interface known to one skilled in the art. USB Interface 108 is an electrical component that is configured to power and provide a data line to hand-held test meter 100.

Micro-controller block 112 also includes a memory sub-block that stores suitable algorithms for the determination of an analyte based on the electrochemical response of an analytical test strip and to also determine a characteristic (e.g., hematocrit) of the introduced bodily fluid sample. Micro-controller block 112 is disposed within housing 110 and can include any suitable micro-controller and/or micro-processer known to those of skill in the art. Suitable micro-controllers include, but are not limited to, micro-controllers available commercially from Texas Instruments (Dallas, Texas, USA) under the MSP430 series of part numbers; from ST MicroElectronics (Geneva, Switzerland) under the STM32F and STM32L series of part numbers; and Atmel Corporation (San Jose, California, USA) under the SAM4L series of part numbers).

Operating range test strip simulation circuit block 114 is in electrical communication with strip port connector 106 (see FIG. 2). Typically, operating range test strip simulation circuit block 114 is configured to be connected and disconnected from electrical contacts of a strip port connector via a user or software controlled switch(s) of the operating range test strip simulation circuit block.

Moreover, operating range test strip simulation circuit block 114 is configured to simulate an inserted electrochemical-based analytical test strip and an operating range of bodily fluid samples applied thereto by sequentially presenting a plurality of electrical loads with each of the plurality of electrical loads configured as (i) a first resistor of predetermined value in series with (ii) a parallel configuration of a second resistor of predetermined value, and a first capacitor of predetermined value. In addition, the plurality of electrical loads spans the operating range of the hand-held test meter with respect to a predetermined bodily fluid sample (such as a whole blood sample) and at least one of an analyte in the bodily fluid sample (for example, glucose) and characteristic (e.g., hematocrit) of the bodily fluid sample.

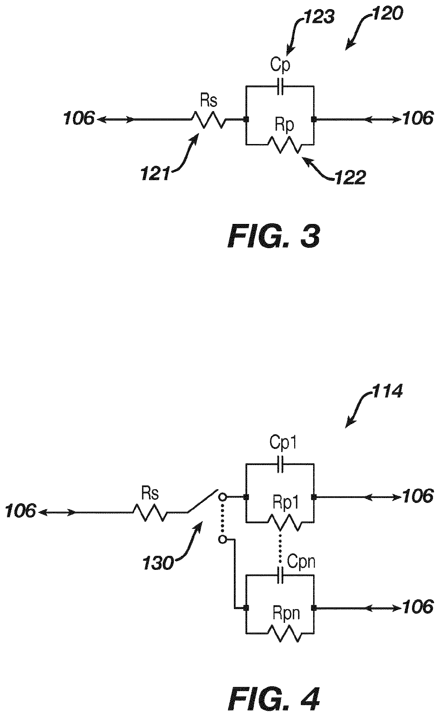

FIG. 3 is a simplified schematic diagram of a single electrical load 120 of operating range test strip simulation circuit block 114. FIG. 4 is a simplified schematic of a configuration of a plurality of electrical loads (i.e., loads 1 through n, where n = any suitable number greater than 1 such as, for example, n = 12 as detailed with regard to Table 1 herein) configured as operating range test strip simulation circuit block 114.

Referring to FIGs. 3 and 4, operating range test strip simulation circuit block 114 includes a plurality of electrical loads, each of the plurality of electrical loads (illustrated by, for example, FIG. 3) is configured as a first resistor 121 of predetermined value in series with a parallel configuration of (i) a second resistor 122 of predetermined value, and a first capacitor 123 of predetermined value. Moreover, operating range test strip simulation circuit block 114 is configured in electrical communication with the micro-controller as noted by the dual-facing arrows in FIGs. 3 and 4. Such electrical communication can be provided, for example, by a direct and/or indirect physical electrical connection between the operating range test strip simulation circuit block and the microcontroller.

Table 1 below provides an exemplary, but non-limiting, tabulation of first resistor, second resistor and first capacitor values for twelve (12) electrical loads as can be employed in an operating range test strip simulation circuit block according to the present invention. In the embodiment of FIG. 4, switch 130 is configured to provide for a sequential presentation of each of the twelve electrical loads. Once apprised of the present invention, one skilled in the art will recognize that the location of switch 130 (or any suitable means for sequentially presenting the plurality of electrical loads) can be placed in alternative locations compared to the depiction of FIG. 4 and/or a plurality of switches can be employed to suitably isolate and sequentially connect the plurality of electrical loads to the SPC. For example, a plurality of electrical loads 120 (see FIG. 3) of predetermined Rₛ, Cₚ, and Rₚ (see, for example, Table 1) could each have dedicated switch(es) connecting them to a single SPC of a hand-held test meter.

The values of Table 1 provide electrical loads that simulate an electrochemical-based test strip with an applied operating range for whole blood samples with hematocrit levels ranging from 29.3% to 55.2%. The predetermined values of Table 1 were experimentally determined for an electrochemical-based analytical test strip with electrical traces having a resistance of 5,100ohm (hence the Rₛ value of 5,100 ohm) and an operating frequency of 75 KHz. The experimental determination included collecting signal phase and magnitude across the operating range and converting these values to resistive and capacitive elements (i.e., Rₚ and Cₚ) through calculation and added to the known strip electrical characteristic (i.e., Rₛ) to build a model of a whole blood sample across the hematocrit range and independent of glucose concentration. The maximum and minimum values of Rₚ and Cₚ for the first nine rows of Table 1 (i.e., n = 1 through 9) include an additional 30% margin as a design and manufacturing guard band for the hand-held test meter electronics. The final 3 rows of Table 1 (i.e., n=10 through 12) are values that were determined in a similar manner as the first nine rows but represent three electrical loads that cover the operating range for control solution measurements. Similar experimental techniques can also be used to determine Rₛ, Rₚ and Cₚ values across the operating for any suitable analyte in a bodily fluid sample such as, for example, glucose in whole blood samples. Therefore, if a plurality of loads corresponding to Table 1 is employed, the operating range test strip simulation circuit block is considered to be further configured to simulate an electrochemical-based analytical test strip inserted into the strip port connector and an operating range of both hematocrit and control solutions applied to the electrochemical-based analytical test strip by sequentially presenting the plurality of loads.

**TABLE 1**

| n | Rₛ (Ohms) | Rₚ (ohms) | Cₚ (pF) |
|---|---|---|---|
| 1 | 5100 | 16000 | 0 |
| 2 | 5100 | 18000 | 5.6 |
| 3 | 5100 | 30000 | 8.2 |
| 4 | 5100 | 33000 | 0 |
| 5 | 5100 | 39000 | 2.2 |
| 6 | 5100 | 51000 | 3.9 |
| 7 | 5100 | 56000 | 0 |
| 8 | 5100 | 59000 | 1.2 |
| 9 | 5100 | 82000 | 2.2 |
| 10 | 5100 | 300000 | 0 |
| 11 | 5100 | 330000 | 1.2 |
| 12 | 5100 | 390000 | 3.3 |

Once apprised of the present disclosure, one skilled in the art will recognize that that embodiments of the present invention can be readily configured by modification of the hand-held test meters described in co-pending Great Britain Patent Application No. 1303616.5, filed on February 28, 2013, which is hereby incorporate in full be reference.

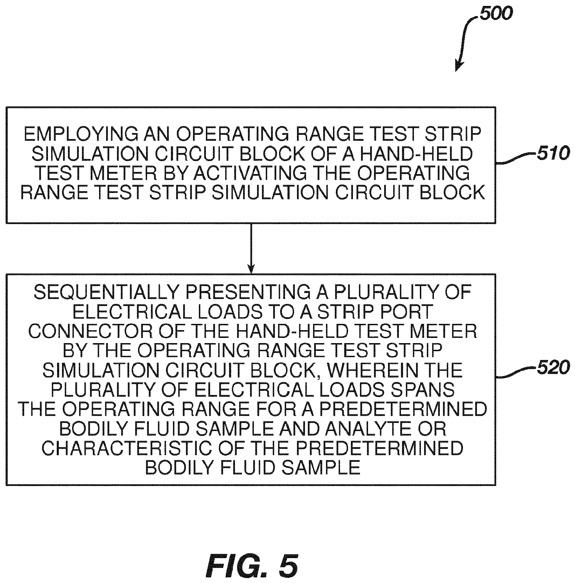

FIG. 5 is a flow diagram depicting stages in a method 500 for employing a hand-held test meter (e.g., hand-held test meter 100 of FIG. 1) for use with an electrochemical-based analytical test strip for the determination of an analyte in, and/or a characteristic of, a bodily fluid sample, according to an embodiment of the present invention. A non-limiting example of such an analyte is glucose in a whole blood sample. A non-limiting example of such a characteristic is hematocrit of a whole blood sample.

Method 500 includes employing an operating range test strip simulation circuit block of a hand-held test meter by activating the operating range test strip simulation circuit block (see step 510 of FIG. 5). Method 500 also includes presenting in a sequential manner, to a port connector of the hand-held test meter by the operating range test strip simulation circuit block, upon the activating of the operating range test strip simulation circuit block, a plurality of electrical loads. Each of the plurality of electrical loads thus presented is configured as a first resistor of predetermined value in series with a parallel configuration of (i) a second resistor of predetermined value and (ii) a first capacitor of predetermined value.

In method 500, the plurality of electrical loads spans the operating range of the hand-held test meter for a predetermined bodily fluid sample and at least one of an analyte in the bodily fluid sample and/or characteristic of the bodily fluid sample.

Once apprised of the present disclosure, one skilled in the art will recognize that methods according to embodiments of the present invention, including method 500, can be readily modified to incorporate any of the techniques, benefits and characteristics of hand-held test meters according to embodiments of the present invention and described herein.

Once apprised of the present disclosure, one skilled in the art will recognize that the meters and methods according to embodiments of the present invention, including method 600, can employ any suitable electrochemical techniques, including those based on Cottrell current measurements, coulometry, amperometry, chronoamperometry, potentiometry, and chronopotentiometry.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that devices and methods within the scope of these claims be covered thereby.

## Claims

1. A hand-held test meter (100) for use with an electrochemical-based analytical test strip (TS) in the determination of an analyte in a bodily fluid sample, the hand-held test meter comprising:
a housing (110);
a micro-controller (112) disposed in the housing;
an operating range test strip simulation circuit block (114) disposed in the housing; and
a strip port connector (106) configured to operationally receive an electrochemical-based analytical test strip;
wherein the operating range test strip simulation circuit block is in electrical communication with the strip port connector; and
wherein the operating range test strip simulation circuit block is configured to simulate an electrochemical-based analytical test strip inserted into the strip port connector and an operating range of bodily fluid samples applied to the electrochemical-based analytical test strip by sequentially presenting:
a plurality of electrical loads (120), each of the plurality of electrical loads configured as:
a first resistor (121) of predetermined value in series with a parallel configuration of only:
a second resistor (122) of predetermined value, and
a first capacitor (123) of predetermined value;
wherein the strip port connector is configured in electrical communication with the micro-controller.

2. A method for employing a hand-held test meter (100) for use with an electrochemical-based analytical test strip (TS) in the determination of an analyte in, or a characteristic of, a bodily fluid sample, the method comprising:
employing an operating range test strip simulation circuit block (114) of a hand-held test meter by activating the operating range test strip simulation circuit block;
presenting in a sequential manner, to a port connector (106) of the hand-held test meter by the operating range test strip simulation circuit block, upon the activating of the operating range test strip simulation circuit block, a plurality of electrical loads (120), each of the plurality of electrical loads configured as:
a first resistor (121) of predetermined value in series with a parallel configuration of only:
a second resistor (122) of predetermined value, and
a first capacitor (123) of predetermined value;
wherein the plurality of electrical loads spans the working range of the hand-held test meter with respect to a predetermined bodily fluid sample and at least one of an analyte in the bodily fluid sample and characteristic of the bodily fluid sample.

3. The method of claim 2 wherein the employing and presenting serves to test operation of the hand-held test meter prior to use of the hand-held test meter for the determination of an analyte.

4. The method of claim 2 or 3 further including:
inserting an electrochemical-based analytical test strip into the hand-held test meter following the presenting and subsequently determining at least one of an analyte in, and a characteristic of, a bodily fluid sample applied to the analytical test strip using a micro-controller (112) of the hand-held test meter.

5. The hand-held test meter of claim 1 or the method of any one of claims 2 - 4 wherein the test strip and applied bodily fluid sample circuit block is configured to simulate a plurality of applied bodily fluid samples across a glucose operating range of the hand-held test meter.

6. The hand-held test meter of claim 1 or the method of any one of claims 2 - 4 wherein the test strip and applied bodily fluid sample circuit block is configured to simulate a plurality of applied bodily fluid samples across a hematocrit operating range of the hand-held test meter.

7. The hand-held test meter of claim 1 or the method of any one of claims 2 - 4 wherein the test strip and applied bodily fluid sample circuit block is configured to simulate a plurality of applied bodily fluid samples across a combined glucose and hematocrit operating range of the hand-held test meter.

8. The hand-held test meter of any one of claims 1 and 5 - 7 or the method of any one of claims 2 - 7 wherein the plurality of electrical loads is twelve electrical loads.

9. The hand-held test meter of any one of claims 1 and 5 - 8 or the method of any one of claims 2 - 8 wherein the first resistor of predetermined value in each of the plurality of electrical loads are essentially identical.

10. The hand-held test meter of any one of claims 1 and 5 - 9 or the method of any one of claims 2 - 9 wherein the plurality of operating loads simulates an operating range that includes a guard band.

11. The hand-held test meter of claim 10 or the method of claim 10 wherein the design and manufacturing guard band is an additional +/- 30% of the maximum and minimum values of the resistance of the second resistor and the capacitance of the first capacitor.

12. The hand-held test meter of any one of claims 1 and 5 - 11 or the method of any one of claims 2 - 11 wherein the first resistor is has a predetermined value of 5,100 ohms, the second resistor has a predetermined value in the range of 16,000 ohms to 390,000 ohms, and the first capacitor has a predetermined value in the range of 0 pF to 6.2 pF or the range of 0 pF to 8.2 pF.

13. The hand-held test meter of any one of claims 1 and 5 - 12 or the method of any one of claims 2 - 12 wherein the plurality of electrical loads share the first resistor.

14. The hand-held test meter of any one of claims 1 and 5 - 13 or the method of any one of claims 2 - 13 wherein the electrochemical-based analytical test strip is an electrochemical-based analytical test strip configured for the determination of glucose in a whole blood bodily fluid sample.

15. The hand-held test meter of any one of claims 1 and 5 - 14 or the method of any of claims 2- 14 wherein the operating range test strip simulation circuit block is further configured to simulate an electrochemical-based analytical test strip inserted into the strip port connector and an operating range of control solutions applied to the electrochemical-based analytical test strip by sequentially presenting the plurality of loads.

## Patentansprüche

1. Handtestmessgerät (100) zur Verwendung mit einem Analyseteststreifen (TS) auf elektrochemischer Basis bei der Bestimmung eines Analyten in einer Körperfluidprobe, wobei das Handtestmessgerät Folgendes umfasst:
ein Gehäuse (110);
eine Mikrosteuerung (112), die in dem Gehäuse angeordnet ist;
einen Betriebsbereichsteststreifensimulation-Schaltkreisblock (114), der in dem Gehäuse angeordnet ist; und
einen Streifenportverbinder (106), der zum einsatzfähigen Empfangen eines Analyseteststreifens auf elektrochemischer Basis konfiguriert ist;
wobei sich der Betriebsbereichsteststreifensimulation-Schaltkreisblock in elektrischer Kommunikation mit dem Streifenportverbinder befindet; und
wobei der Betriebsbereichsteststreifensimulation-Schaltkreisblock zum Simulieren eines Analyseteststreifens auf elektrochemischer Basis, der in den Streifenportverbinder eingefügt ist, und eines Betriebsbereichs von Körperflüssigkeitsproben, die auf den Analyseteststreifen auf elektrochemischer Basis aufgebracht sind, durch sequenzielles Präsentieren von Folgendem konfiguriert ist:
mehreren elektrischen Lasten (120), wobei jede der mehreren elektrischen Lasten konfiguriert ist als:
ein erster Widerstand (121) mit einem vorbestimmten Wert in Reihe mit einer Parallelkonfiguration aus nur:
einem zweiten Widerstand (122) mit einem vorbestimmten Wert, und
einem ersten Kondensator (123) mit einem vorbestimmten Wert;
wobei der Streifenportverbinder in elektrischer Kommunikation mit der Mikrosteuerung konfiguriert ist.

2. Verfahren zum Einsetzen eines Handtestmessgeräts (100) zur Verwendung mit einem Analyseteststreifen (TS) auf elektrochemischer Basis bei der Bestimmung eines Analyten in, oder einer Charakteristik von, einer Körperfluidprobe, wobei das Verfahren Folgendes umfasst:
Einsetzen eines Betriebsbereichsteststreifensimulation-Schaltkreisblocks (114) eines Handtestmessgeräts durch Aktivieren des Betriebsbereichsteststreifensimulation-Schaltkreisblocks;
Präsentieren, für einen Portverbinder (106) des Handtestmessgeräts durch den Betriebsbereichsteststreifensimulation-Schaltkreisblocks, mehrerer elektrischer Lasten (120) auf eine sequenzielle Weise beim Aktivieren des Betriebsbereichsteststreifensimulation-Schaltkreisblock, wobei jede der mehreren elektrischen Lasten konfiguriert ist als:
ein erster Widerstand (121) mit einem vorbestimmten Wert in Reihe mit einer Parallelkonfiguration aus nur:
einem zweiten Widerstand (122) mit einem vorbestimmten Wert, und
einem ersten Kondensator (123) mit einem vorbestimmten Wert;
wobei die mehreren elektrischen Lasten den Arbeitsbereich des Handtestmessgeräts mit Bezug auf eine vorbestimmte Körperfluidprobe und einen Analyten in der Körperfluidprobe und/oder Charakteristiken der Körperfluidprobe aufspannen.

3. Verfahren nach Anspruch 2, wobei das Einsetzen und Präsentieren einem Testbetrieb des Handtestmessgeräts vor einer Verwendung des Handtestmessgeräts für die Bestimmung eines Analyten dient.

4. Verfahren nach Anspruch 2 oder 3, das ferner Folgendes beinhaltet:
Einfügen eines Analyseteststreifens auf elektrochemischer Basis in das Handtestmessgerät anschließend an das Präsentieren und anschließend Bestimmen eines Analyten und/oder einer Charakteristik einer Körperfluidprobe, die auf den Analyseteststreifen aufgebracht ist, unter Verwendung einer Mikrosteuerung (112) des Handtestmessgeräts.

5. Handtestmessgerät nach Anspruch 1 oder Verfahren nach einem der Ansprüche 2-4, wobei der Teststreifen- und-aufgebrachte-Körperfluidprobe-Schaltkreisblock zum Simulieren mehrerer aufgebrachter Körperfluidproben über einen Glucosebetriebsbereich des Handtestmessgeräts konfiguriert ist.

6. Handtestmessgerät nach Anspruch 1 oder Verfahren nach einem der Ansprüche 2-4, wobei der Teststreifen- und-aufgebrachte-Körperfluidprobe-Schaltkreisblock zum Simulieren mehrerer aufgebrachter Körperfluidproben über einen Hämatokritbetriebsbereich des Handtestmessgeräts konfiguriert ist.

7. Handtestmessgerät nach Anspruch 1 oder Verfahren nach einem der Ansprüche 2-4, wobei der Teststreifen- und-aufgebrachte-Körperfluidprobe-Schaltkreisblock zum Simulieren mehrerer aufgebrachter Körperfluidproben über einen kombinierten Glucose- und Hämatokritbetriebsbereich des Handtestmessgeräts konfiguriert ist.

8. Handtestmessgerät nach einem der Ansprüche 1 und 5-7 oder Verfahren nach einem der Ansprüche 2-7, wobei die mehreren elektrischen Lasten zwölf elektrische Lasten sind.

9. Handtestmessgerät nach einem der Ansprüche 1 und 5-8 oder Verfahren nach einem der Ansprüche 2-8, wobei der erste Widerstand mit einem vorbestimmten Wert in jeder der mehreren elektrischen Lasten im Wesentlichen identisch ist.

10. Handtestmessgerät nach einem der Ansprüche 1 und 5-9 oder Verfahren nach einem der Ansprüche 2-9, wobei die mehreren Betriebslasten einen Betriebsbereich simulieren, der ein Schutzband beinhaltet.

11. Handtestmessgerät nach Anspruch 10 oder Verfahren nach Anspruch 10, wobei das Gestaltungs- und Herstellungsschutzband zusätzliche +/- 30 % der maximalen Werte des Widerstandswertes des zweiten Widerstands und der Kapazität des ersten Kondensators sind.

12. Handtestmessgerät nach einem der Ansprüche 1 und 5-11 oder Verfahren nach einem der Ansprüche 2-11, wobei der erste Widerstand einen vorbestimmten Wert von 5.100 Ohm aufweist, der zweite Widerstand einen vorbestimmten Wert in dem Bereich von 16.000 Ohm bis 390.000 Ohm aufweist und der erste Kondensator einen vorbestimmten Wert in dem Bereich von 0 pF bis 6,2 pF oder dem Bereich von 0 pF bis 8,2 pF aufweist.

13. Handtestmessgerät nach einem der Ansprüche 1 und 5-12 oder Verfahren nach einem der Ansprüche 2-12, wobei die mehreren elektrischen Lasten den ersten Widerstand teilen.

14. Handtestmessgerät nach einem der Ansprüche 1 und 5-13 oder Verfahren nach einem der Ansprüche 2-13, wobei der Analyseteststreifen auf elektrochemischer Basis ein Analyseteststreifen auf elektrochemischer Basis ist, der für die Bestimmung von Glucose in einer Vollblutkörperfluidprobe konfiguriert ist.

15. Handtestmessgerät nach einem der Ansprüche 1 und 5-14 oder Verfahren nach einem der Ansprüche 2-14, wobei der Betriebsbereichsteststreifensimulation-Schaltkreisblock ferner zum Simulieren eines Analyseteststreifens auf elektrochemischer Basis, der in den Streifenportverbinder eingefügt ist, und eines Betriebsbereichs von Kontrolllösungen, die auf den Analyseteststreifen auf elektrochemischer Basis aufgebracht sind, durch sequenzielles Präsentieren der mehreren Lasten konfiguriert ist.

## Revendications

1. Appareil de mesure portable (100) destiné à être utilisé avec une bande d'essai analytique à base électrochimique pour la détermination d'un analyte dans un échantillon de fluide corporel, l'appareil de mesure portable comprenant :
un boîtier (110) ;
un microcontrôleur (112) disposé dans le boîtier ;
un bloc de circuit de simulation de bande pour l'essai de la plage de fonctionnement (114) disposé dans le boîtier ; et
un connecteur de port de bande (106) configuré pour recevoir de manière opérationnelle une bande d'essai analytique à base électrochimique ;
dans lequel le bloc de circuit de simulation de bande pour l'essai de la plage de fonctionnement est en communication électrique avec le connecteur du port de bande ; et
dans lequel le bloc de circuit de simulation de bande pour l'essai de la plage de fonctionnement est configuré pour simuler une bande d'essai analytique à base électrochimique introduite dans le connecteur du port de bande et une plage de fonctionnement des échantillons de fluide corporel appliqués à la bande d'essai analytique à base électrochimique en présentant en séquence :
une pluralité de charges électriques (120), chacune de la pluralité des charges électriques étant configurée comme :
une première résistance (121) de valeur prédéterminée en série avec une configuration en parallèle comprenant seulement :
une seconde résistance (122) de valeur prédéterminée et
un premier condensateur (123) de valeur prédéterminée ;
dans lequel le connecteur du port de bande est configuré en communication électrique avec le microcontrôleur.

2. Procédé d'utilisation d'un appareil de mesure portable (100) destiné à être utilisé avec une bande d'essai analytique à base électrochimique lors de la détermination d'un analyte dans un échantillon de fluide corporel ou d'une caractéristique de celui-ci, le procédé comprenant les étapes consistant à :
utiliser un bloc de circuit de simulation de bande pour l'essai de la plage de fonctionnement (114) d'un appareil de mesure portable en activant le bloc de circuit de simulation de bande pour l'essai de la plage de fonctionnement ;
présenter en séquence, à l'activation du bloc de circuit de simulation de bande pour l'essai de la plage de fonctionnement, à un connecteur de port (106) de l'appareil de mesure portable à l'aide du bloc de circuit de simulation de bande pour l'essai de la plage de fonctionnement, une pluralité de charges électriques (120), chacune de la pluralité des charges électriques étant configurée comme suit :
une première résistance (121) de valeur prédéterminée en série avec une configuration en parallèle comprenant seulement :
une seconde résistance (122) de valeur prédéterminée et
un premier condensateur (123) de valeur prédéterminée ;
dans lequel la pluralité des charges électriques couvre la plage de travail de l'appareil de mesure portable par rapport à un échantillon de fluide corporel prédéterminé et à au moins un analyte dans l'échantillon de fluide corporel ou une caractéristique de l'échantillon de fluide corporel.

3. Procédé selon la revendication 2, dans lequel l'utilisation et la présentation servent à tester le fonctionnement de l'appareil de mesure portable avant l'utilisation de l'appareil de mesure portable pour la détermination d'un analyte.

4. Procédé selon la revendication 2 ou 3 comprenant en outre les étapes consistant à :
introduire une bande d'essai analytique à base électrochimique dans l'appareil de mesure portable après la présentation et déterminer ensuite au moins un analyte dans un échantillon de fluide corporel et une caractéristique d'un échantillon de fluide corporel appliqué à la bande d'essai analytique à l'aide d'un microcontrôleur (112) de l'appareil de mesure portable.

5. Appareil de mesure portable selon la revendication 1 ou procédé selon l'une quelconque des revendications 2 à 4, dans lequel la bande d'essai et le bloc de circuit d'échantillon de fluide corporel appliqué sont configurés pour simuler une pluralité d'échantillons de fluide corporel appliqués sur une plage de fonctionnement pour la détermination du glucose de l'appareil de mesure portable.

6. Appareil de mesure portable selon la revendication 1 ou procédé selon l'une quelconque des revendications 2 à 4, dans lequel la bande d'essai et le bloc de circuit d'échantillon de fluide corporel appliqué sont configurés pour simuler une pluralité d'échantillons de fluide corporel appliqués sur une plage de fonctionnement pour la détermination de l'hématocrite de l'appareil de mesure portable.

7. Appareil de mesure portable selon la revendication 1 ou procédé selon l'une quelconque des revendications 2 à 4, dans lequel la bande d'essai et le bloc de circuit d'échantillon de fluide corporel appliqué sont configurés pour simuler une pluralité d'échantillons de fluide corporel appliqués sur une plage de fonctionnement combinée du glucose et de l'hématocrite dans l'appareil de mesure portable.

8. Appareil de mesure portable selon l'une quelconque des revendications 1 et 5 à 7, ou procédé selon l'une quelconque des revendications 2 à 7, dans lequel la pluralité des charges électriques est de douze charges électriques.

9. Appareil de mesure portable selon l'une quelconque des revendications 1 et 5 à 8, ou procédé selon l'une quelconque des revendications 2 à 8, dans lequel la première résistance de valeur prédéterminée dans chacune de la pluralité des charges électriques est essentiellement la même.

10. Appareil de mesure portable selon l'une quelconque des revendications 1 et 5 à 9, ou procédé selon l'une quelconque des revendications 2 à 9, dans lequel la pluralité des charges de fonctionnement simule une plage de fonctionnement qui comprend une bande de garde.

11. Appareil de mesure portable selon la revendication 10 ou procédé selon la revendication 10, dans lequel la bande de garde de conception et de fabrication est de +/- 30% supplémentaires aux valeurs maximales et minimales de la résistance de la seconde résistance et de la capacité du premier condensateur.

12. Appareil de mesure portable selon l'une quelconque des revendications 1 et 5 à 11, ou procédé selon l'une quelconque des revendications 2 à 11, dans lequel la première résistance a une valeur prédéterminée de 5 100 ohms, la seconde résistance a une valeur prédéterminée dans la plage de 16 000 ohms à 390 000 ohms et le premier condensateur a une valeur prédéterminée dans la plage de 0 pF à 6,2 pF ou dans la plage de 0 pF à 8,2 pF.

13. Appareil de mesure portable selon l'une quelconque des revendications 1 et 5 à 12, ou procédé selon l'une quelconque des revendications 2 à 12, dans lequel la pluralité des charges électriques partage la première résistance.

14. Appareil de mesure portable selon l'une quelconque des revendications 1 et 5 à 13, ou procédé selon l'une quelconque des revendications 2 à 13, dans lequel la bande d'essai analytique à base électrochimique est une bande d'essai analytique à base électrochimique configurée pour la détermination du glucose dans un échantillon de fluide corporel de sang total.

15. Appareil de mesure portable selon l'une quelconque des revendications 1 et 5 à 14, ou procédé selon l'une quelconque des revendications 2 à 14, dans lequel le bloc de circuit de simulation de bande pour l'essai de la plage de fonctionnement est en outre configuré pour simuler une bande d'essai analytique à base électrochimique introduite dans le connecteur du port de bande et une plage de fonctionnement des solutions de contrôle appliquées à la bande d'essai analytique à base électrochimique en présentant en séquence la pluralité des charges.
